# EUROPEAN PATENT APPLICATION

(11) **EP 2 036 987 A1**
(43) Date of publication of application: **18.03.2009**
(21) Application number: 07018233.2
(22) Date of filing: 17.09.2007
(51) Int. Cl.: C12Q 1/68

(54) **Molecular markers for tumor cell content in tissue samples**

(71) Applicant: Siemens Healthcare Diagnostics GmbH, 65760 Eschborn (DE)
(72) Inventor: Hennig, Guido, Dr., 50859 Köln (DE); Kronenwett, Ralf, Dr., 40237 Düsseldorf (DE); von Thörne, Christian, Dr., 42659 Solingen (DE)
(74) Representative: Maier, Daniel Oliver

(57) **Abstract**

The invention relates to a method of determining the tumor cell content of a sample from a patient suffering from or at risk of developing a neoplastic disease, comprising the steps of: (a) isolating total RNA from the sample and (b) determining the expression level of at least one tumor cell content (TCC) marker gene. A selection of highly informative TCC marker genes is given.

## Description

The present invention relates to methods for determining the tumor cell content in patient samples.

Cancer is a heterogeneous disease and is routinely classified according to morphology, immunohistochemistry and clinical stage. Currently the assessment of prognosis and prediction of response to a specific therapy is based on clinical and pathological data. Recent studies suggest that gene expression profiling using microarrays or (simplex or multiplex) quantitative PCR (qPCR) may help in improving prediction of prognosis and treatment response and thus improving patienttailored therapy. In gene expression studies, routinely non-or macro-dissected tumor material from surgery or needle biopsies is used. However, tissue samples are heterogeneous with respect to tumor cell content (TCC), proportions of connective tissue, vascular and inflammatory components, or other constituents. The composition of tissue varies between tumor types, sampling processes and also across a single tumor. In many cases, a sample will contain both tumor cells as well as adjacent normal tissue and the proportion of tumor cells, i.e. tumor cell content, may vary greatly from sample to sample.

Tumor cell content is an important variable in gene expression analysis because assessment of a gene expression profile of a biological sample containing tumor cells depends on the total tumor cell content and requires a minimum amount of tumor cell content to be present in a given sample. Tumor cell content is a measure or indication of the amount, relative amount, or percentage of tumor material in sample. It can be expressed e.g. as percentage of tumorous cells vs. non-tumorous cells, as rating or classification, e.g. ranging from "no tumorous material present" to "only tumorous material present", or the like. A parameter indicating tumor cell content of a sample could also be a valuable tool for normalizing gene expression data and will facilitate the comparison between different sets of data derived from different assays or experience. Furthermore, a parameter indicative of total tumor cell content of a given sample could in itself be used as prognostic or predictive marker, e.g. when the tumor cell content of a discrete sample such as a given volume of blood or an entire lymph node is assessed, and could aid the clinician in choosing an appropriate therapy. A third interesting application is the use of the estimate of the tumor cell content as an internal control for assay quality control.

Currently, tumor cell content is estimated by a pathologist through visual inspection of histological samples. However, there is no internationally excepted standard for the definition of tumor cell content. Accepted tumor cell content is routinely estimated by calculation of the proportion of tumor cells in comparison to normal cells or calculating the proportion of tumor area including inflammatory cells and stromal components compared to non-tumor area on histological sections. These methods should only be carried-out by experienced pathologists and are prone to variations between the assessments of individual pathologists. The classification of DCIS (**D**uctal **C**arcinoma **I**n **S**itu, i.e. non-invasive part of tumor) components to the tumor or non-tumor part also varies between pathologists and is not standardized.

There are numerous approaches to detect tumor cells by measuring mRNA concentration of tumor associated genes, for example, in neuroblastoma (Cheung et al., Cancer 2004;101:2303-8, Cheung et al. Cancer 2002;94:3042-8), prostate cancer ( Schostak et al., BMC Urology 2006, 6:7), gastrointestinal cancer (Cui et al., World J Gastroenterol, 2001;7(3):381-386) or other cancer or neoplastic diseases.

However, there are no validated gene expression markers for a molecular assessment of tumor cell content of a sample so far.

Definitions

The term "neoplastic disease", "neoplastic region", or "neoplastic tissue" refers to a tumorous tissue including carcinoma (e.g. carcinoma in situ, invasive carcinoma, metastasis carcinoma) and pre-malignant conditions, neomorphic changes independent of their histological origin.

The term "cancer" is not limited to any stage, grade, histomorphological feature, aggressivity, or malignancy of an affected tissue or cell aggregation. In particular, solid tumors, malignant lymphoma and all other types of cancerous tissue, malignancy and transformations associated therewith, lung cancer, ovarian cancer, cervix cancer, stomach cancer, pancreas cancer, prostate cancer, head and neck cancer, renal cell cancer, colon cancer or breast cancer are included. The terms "neoplastic lesion" or "neoplastic disease" or "neoplasm" or "cancer" are not limited to any tissue or cell type. They also include primary, secondary, or metastatic lesions of cancer patients, and also shall comprise lymph nodes affected by cancer cells or minimal residual disease cells either locally deposited or freely floating throughout the patient's body.

In the context of the present invention, the term "sample" refers to a sample obtained from a patient. The sample may be of any biological tissue or fluids. Such samples include, but are not limited to, blood, blood cells (e.g. lymphocytes, leucocytes, macrophages etc.), stool sample, urine, peritoneal fluid, pleural fluid, cells therefrom, tissue, biopsy samples, cell-containing body fluids, fresh-frozen tissue material, tumor tissue obtained in surgery, embedded tissue samples (e.g. histological sections), smear samples, lavage samples, nipple discharge, serum, plasma, lymph, ascites, and other body fluids.

The term "expression level" refers to a determined level of gene expression. This may be a determined level of gene expression compared to a reference gene (e.g. a housekeeping gene), to a computed average expression value (e.g. in DNA chip analysis), or to another informative gene without the use of a housekeeping gene. The expression level of a gene may be measured directly, e.g. by obtaining a signal wherein the signal strength is correlated to the amount of mRNA transcripts of that genes or it may be obtained indirectly at a protein level, e.g. by ELISA, RIA, IHC or CISH methods. Measurements may also directly be obtained using a competitive reaction to a reference sample.

The term "prediction" refers to an individual assessment of malignancy of a tumor or the expected survival of a patient, if a patient is treated with a given therapy. The term "prognosis" relates to an individual assessment of the malignancy of a tumor or the expected survival rate of a patient, if the patient remains untreated. The term "diagnosis" refers to an individual assessment of the type of cancer or neoplastic disease which a patient is afflicted by.

The term "marker gene" refers to a differentially expressed gene whose expression pattern is (as a single gene or in combination with other markers) indicative of the presence of a specific phenotype, condition or pathologic condition, e.g. neoplastic disease or cancer.

The term "internal standard gene" refers to a gene which is constantly and stably expressed in a wide variety of tissues or cells, e.g. housekeeping genes such as GAPDH, β-Aktin, β-2-Mikroglobulin, ribosomal RNA, and others and combinations of a plurality thereof.

The term "primer", "primer pair", "probe", or "primer-probe set", shall have ordinary meaning of these terms which is known to the person skilled in the art of molecular biology. In a preferred embodiment of the invention "primer", "primer pair" and "probes" refer to oligonucleotide or polynucleotide molecules with a sequence identical to, complementary to, homologues of, or homologous to regions of the target molecule or target sequence which is to be detected or quantified, such that the primer, primer pair or probe can specifically bind to the target molecule, e.g. target nucleic acid, gene, transcript, peptide, polypeptide, or protein to be detected or quantified. Probe technologies used for kinetic or real time PCR applications could be e.g. Taqman, Scorpions, Dual Hybridisation Probes, Amplifluor or Minor Groove Binders.

The term "complementary" or "sufficiently complementary" means a degree of complementarity which is - under given assay conditions - sufficient to allow the formation of a binding complex of a primer or probe to a target molecule. Assay conditions which have an influence of binding of probe to target include temperature, solution conditions, such as composition, pH, ion concentrations, etc. as is known to the skilled person.

The term "hybridization based method", as used herein, refers to methods imparting a process of combining complementary, single-stranded nucleic acids or nucleotide analogues into a single double stranded molecule. Nucleotides or nucleotide analogues will bind to their complement under normal conditions, so two perfectly complementary strands will bind to each other readily. In bioanalytics, very often labeled, single stranded probes are used in order to find complementary target sequences. If such sequences exist in the sample, the probes will hybridize to said sequences which can then be detected due to the label. Other hybridization based methods comprise microarray and/or biochip methods. Therein, probes are immobilized on a solid phase, which is then exposed to a sample. If complementary nucleic acids exist in the sample, these will hybridize to the probes and can thus be detected. Hybridization is dependent on target and probe (e.g. length of matching sequence, GC content, binding affinity of antibody and antigen) and hybridization conditions (temperature, solvent, pH, ion concentrations, presence of denaturing agents, etc.).

The term "array" refers to an arrangement of addressable locations on a device, e.g. a chip device. The number of locations can range from several to at least hundreds or thousands. Each location represents an independent reaction site. Arrays include, but are not limited to, nucleic acid arrays, protein arrays and antibody arrays. A "nucleic acid array" refers to an array containing nucleic acid probes, such as oligonucleotides, polynucleotides or larger portions of genes. The nucleic acid on the array is preferably single-stranded. A "microarray" refers to a biochip or biological chip, i.e. an array of regions having a density of discrete regions with immobilized probes of at least about 100/cm^{2.}

A "PCR-based method" refers to methods comprising a polymerase chain reaction (PCR). This is a method of exponentially amplifying nucleic acids, e.g. DNA by enzymatic replication in vitro. For RNA amplification, one can use reverse transcription as a first step. PCR-based methods comprise quantitative PCR which is particularly suited for the analysis of expression levels.

The term "determining a protein level" refers to any method suitable for quantifying the amount, amount relative to a standard or concentration of a given protein in a sample. Commonly used methods to determine the amount of a given protein are e.g. ELISA, RIA, Western blot with densitometric quantification, IHC, CISH etc.

The term "predicting a clinical outcome" in the context of the present invention encompasses both the "prognosis" (prediction of a clinical outcome of neoplastic disease in the absence of a treatment as defined above) and "prediction" (clinical outcome of neoplastic disease in the presents of a given treatment as defined above).

The term "mode of treatment", "therapy mode", "regimen" or "therapy regimen" refers to a timely sequential or simultaneous administration of surgical measures, anti-tumor, and/or anti vascular, and/or immune stimulating, and/or blood cell proliferative agents, and/or radiation therapy, and/or hyperthermia, and/or hypothermia for cancer therapy. The composition of the corresponding treatment protocols may vary in the dose of the single agent, timeframe of application and frequency of administration within a defined therapy window. Currently various combinations of various drugs and/or physical methods, and various schedules are under investigation.

The phrase "tumor response", "therapeutic success", "response", or "response to therapy" refers, in the adjuvant chemotherapeutic setting, to the observation of a defined tumor-free or recurrence-free survival time (e.g. 2 years, 4 years, 5 years, 10 years). This time period of disease-free survival may vary among the different tumor entities but is sufficiently longer than the average time period in which most of the recurrences appear. In a neo-adjuvant therapy modality, response may be monitored by measurement of tumor shrinkage and regression due to apoptosis and necrosis of the tumor mass.

The term "recurrence" or "recurrent disease" includes the reappearance of distant metastasis that can appear even many years after the initial diagnosis and therapy of a tumor, or local events such as infiltration of tumor cells into regional lymph nodes, or occurrence of tumor cells at the same site and organ of origin within an appropriate time frame.

### Object of the invention:

It is an object of the invention to provide a method for determining the tumor cell content of a sample from a patient suffering from a neoplastic disease or at risk of developing a neoplastic disease.

It is a further object of the invention to provide a method of predicting a clinical outcome for a patient afflicted with a neoplastic disease.

### Summary of the invention

The objects of the present invention are attained by providing the methods of claims 1 and 2 and by providing the kit of claim 9.

The present invention is predicated on the identification of a group of genes, herein referred to as "tumor cell content marker genes" or "TCC marker genes" which allow the assessment of tumor cell content of a given sample. The invention is further predicated on the identification of certain chromosomal locations which contain hot spots of highly informative tumor cell content marker genes.

These tumor cell content marker genes are defined and identified as follows:
1. A gene selected from the group consisting of the genes identified in table 1 below.
2. A gene identified by the method described below and mapping to a chromosomal location selected from the group consisting of 1q22-32, 2p14-22, 3p11-25, 3q11-27, 4q12-28, 7q21-31, 9q31-34, 11q13-23, 12q13-14, 17q21-24.

Tumor cell content markers genes according to (2.) can be identified as follows:
i.) Tumor cell content of patient samples is determined as percentage of tumor cells or percentage of tumor area, mass, or volume in the sample. Tumor cell content can be e.g. analyzed or determined by visual analysis.
ii.) Samples are discretized into samples with low tumor cell content and high tumor content, e.g. samples with tumor cell content above or below a threshold value or e.g. above an upper threshold value or below a lower threshold value. To improve statistic significance in the following step, optionally samples with indeterminate or medium tumor cell content may be disregarded for further analysis.
iii.) RNA is isolated from the sample. This may be achieved e.g. by using one histological section for visual analysis and using the adjacent section for RNA isolation.
iv.) The expression levels of genes expected to be candidates as tumor cell content marker genes is determined in a plurality of samples, e.g. samples from different patients.
v.) Informative genes for separation of both groups are identified by a statistical method, such as e.g. t-Test, Wilcoxon-Mann-Whitney test, Kolmogorov-Smirnov test or in general any supervised on unsupervised clustering approach or classification strategy, and genes mapping to the chromosomal locations under (2.) are selected as tumor cell content (TCC) marker genes.

The term "visual analysis" relates to the visual inspection of a sample, in particular a tissue sample, such as a histologic section, embedded section, cryo section or the like, which sample may be treated so as to highlight morphologic structures or highlight tumor cells, thus allowing to differentiate tumor cells from non-tumor cells. The highlighting or labeling of cells may be performed by any suitable method to allow differentiation of tumor cells and non-tumor cells, such as hematoxylin and eosin stain (HE staining), immunohistochemical staining or the like. Visual inspection is preferably performed by a person trained in the art of histology, such as pathologists, histotechnicians, histology technicians, histology technologists, medical scientists, medical laboratory technicians or biomedical scientists. As an alternative, this analysis may be performed by a suitable imaging analysis software which can differentiate tumor cells or tumor tissue from non-tumor cells or non-tumor tissue.

Specifically, TCC marker genes can be obtained e.g. by the following method: Total RNA from a number (n ≥ 10, n ≥ 50, or n ≥ 100) of patient samples is isolated and tumor cell content of these samples is con-comitantly analysed by tumor cell specific staining of histologic sections and visual analysis according to the state of the art. Samples are then identified as having a tumor cell content between 0% and 100% by visual analysis and the samples are discretized into 2 groups: tumor cell content < 33% (low tumor cell content) and tumor cell content > 66% (high tumor cell content), or alternatively tumor cell content < 25% (low tumor cell content) and tumor cell content > 75% (high tumor cell content). Expression analysis of low TCC samples and high TCC samples is performed with the corresponding RNA samples on a whole-genome micro-array chip (for example Affymetrix U133A micro-array) and best informative genes for univariate separation of both groups are identified by t-Test statistics and genes mapping to the chromosomal locations lifted under (2.) are selected as tumor cell content (TCC) marker genes.

Determining the tumor cell content by measuring the expression level of a plurality of TCC marker genes and forming a new score based on the combined (e.g. average) expression level value of the plurality of TCC marker genes allows an even better separation. This points to the fact that using a combination of a plurality of TCC marker genes yields even more reliable results for tumor cell content of a sample.

To validate a combination of a plurality of TCC marker genes, multivariate analysis can be used for discovery, e.g. supervised methods such as principal component analysis (PCA), singular value decomposition (SVD) or standard cluster algorithms, such as, for example, k-nearest neighbors (kNN), support vector machines (SVM), logistic regression, naive Bayes classification, or supervised methods such as projection methods or standard cluster algorithms. Weighted voting, or majority voting may be used in both cases.

The inventive method of determining the tumor cell content of a sample comprises the steps of:
- isolating total RNA from the sample; and
- determining the expression level of at least one tumor cell content marker gene which TCC marker gene is defined as above.

According to an aspect of the invention, the expression level of 2 to 20, 2 to 10, 2 to 5, or 3 different tumor cell content marker genes may be determined in the same assay or test.

According to an aspect of the present invention, the expression level of at least one tumor cell content marker gene is determined in relation to the expression level of an internal standard gene.

According to aspect of the present invention, the expression level may be determined by one or more of the following methods:
a) a hybridization-based method;
b) a PCR-based method;
c) determining the protein level; and
d) an array-based method.

According to another aspect of the present invention, in an additional step, the tumor cell content of the sample may be correlated with patient data, wherein that data is selected from the group consisting of gene expression level of a further marker gene which is not a TCC marker gene as defined above, etiopathology data, clinical symptoms, anamnesis data and/or data concerning the therapeutic regimen.

The term "etiopathology" relates to the course of disease, its duration, its clinical symptoms, and its outcome.

The term "anamnesis" relates to patient data obtained by a physician or other health care professional by asking specific questions of the patient or a person who knows the patient and can give suitable information, with the aim of obtaining the information useful in arriving at a diagnosis and providing medical care to the patient. This kind of information is referred to as "symptoms", in contrast with clinical parameters. The term "clinical parameters" refers to parameters obtained by examination of the patient or patient samples, such as, but not limited to, vital parameters (e.g. pulse, blood pressure), results obtained by in vitro-diagnostic tests (e.g. clinical chemistry), results obtained by imaging methods (e.g. computed tomography, magnetic resonance imaging, x-ray, ultrasound) and others.

In order to allow comparisons between different samples, standard sample sizes or types of sample may be defined, such as a given volume of a body fluid (e.g. blood) or tissue, a given mass of body fluid or tissue, a defined organ or a defined part of an organ, e.g. lymph node, cross-section of a lymph node with defined section area and/or section thickness, or the like.

According to an embodiment of the present invention, the sample contains breast cancer cells or is suspected of containing breast cancer cell or is a sample of a patient afflicted with breast cancer or at risk of being afflicted by breast cancer.

According to an embodiment of the present invention, there is provided a method of quantifying an expression level of a gene in a biological sample, wherein the expression level of said gene in the sample is determined in relation to the tumor cell content as determined according to the method described above.

According to an aspect of the present invention, there is provided a method of predicting a clinical outcome for a patient afflicted with a neoplastic disease or suspected of being afflicted with a neoplastic disease, that method comprising the steps of:
a) obtaining a biological sample from that patient;
b) predicting from the tumor cell content of that sample as determined by any of the methods of the invention as described above, a therapeutic success for a plurality of individual modes of treatment; and
c) selecting a mode of treatment which is predicted to be successful in step (b)

For predicting the clinical outcome for a patient afflicted with a neoplastic disease or suspected of being afflicted with a neoplastic disease, it is further possible to determine an expression level of one or more further genes in the biological sample in relation to the tumor cell content.

The mode of treatment is selected in accordance with the predicition obtained by the inventive method and is selected in view of an optimal tumor response, or therapeutic succession order to improve or increase tumor-free or recurrence-free survival time.

Determining the expression level of a further gene (which may be done by a single measurement or by 2-fold, 3-fold or multiple replicate measurements) in relation to the tumor cell content can be achieved by e.g. determining in a suitable assay format or by a suitable method a quantifiable first signal indicative of the amount of mRNA of said further gene and determining a quantifiable second signal indicative of the tumor cell content according to the method of the invention and determining the difference between the first and second signal or forming the quotient (ratio) of first to second signal or performing some other suitable logic operation to compare the first and second signal. Determining the expression level of the further gene in relation to the tumor cell content allows a normalization of experimental results and can be used to reduce variation of results between different assays.

According to the invention, there is further provided a kit useful for carrying out any of the methods of the invention as described above comprising at least:
- a primer pair/or a probe each having a sequence sufficiently complementary to a tumor cell content marker gene selected from the group consisting of genes identified in table 1 and/or a tumor cell content marker gene mapping to a chromosomal location selected from the group consisting of 1q22-32, 2p14-22, 3p11-25, 3q11-27, 4q12-28, 7q21-31, 9q31-34, 11q13-23, 12q13-14, 17q21-24; and/or at least one antibody directed against a protein encoded by a tumor cell content marker gene as defined above.

The methods of the present invention rely on the use of a few highly informative genes whose expression allows the distinction between high and low tumor cell content in a given biological sample. These genes are referred to as "tumor cell content marker genes" and are defined as described above. The expression of the tumor cell content marker genes can be internally used in an expression profile assay to recover the content of tumor cells in a mixed sample, e.g. a fresh-frozen sample or a fixed tissue sample. The tumor cell content marker genes can be used alone or in combination with each other. The combination of TCC marker genes by an algorithm or by majority vote, for example, can stabilize a classifier for estimation of tumor cell content.

The invention will now be described in more detail in connection with the following examples and attached Figures, in which:
Figures 1 and 2 show a table listing tumor cell marker genes according to the present invention;
Figures 3, 4 and 5 show histograms depicting the separation of high tumor cell content and low tumor cell content samples according the expression level of 3 exemplary tumor cell content marker genes, IGFBP7, PPP1R12B, and SERP1, respectively.

Measurement of tumor cell content marker gene expression can be performed on the mRNA level by any suitable method, e.g. qPCR or gene expression array platforms, including commercially available platforms, such as Affymetrix, Illumina, Luminex, planar wave guide, electrochemical microarray chips, microarray chips with optical, magnetic, electrochemical, or gravimetric detection systems and others or on a protein level by immunological techniques such as ELISA.

The term "planar waveguide" relates to methods for detection of the presence or amount of a target molecule by using a planar wave guide detector which emits an evanescent field in order to detect the binding of a labeled target molecule, such as e.g. described in WO200113096-A1.

The term "optical detection" relates to methods which detect the presence or amount of a target molecule through a change in optical properties, e.g. fluorescence, absorption, reflectance, chemiluminescence, as is well known in the art.

The term "magnetic detection" relates to methods which detect the presence or amount of a target molecule through a change in magnetic properties, e.g. through the use of XMR sensors, GMR sensors or the like.

The term "electrochemical detection" relates to methods which make use of an electrode system to which molecules, particularly biomolecules like proteins, nucleic acids, antigens, antibodies and the like, bind under creation of a detectable signal. Such methods are for example disclosed in WO0242759, WO200241992 and WO2002097413, the content of which is incorporated by reference herein. These detectors comprise a substrate with a planar surface and electrical detectors which may adopt, for example, the shape of interdigital electrodes or a two dimensional electrode array. These electrodes carry probe molecules, e.g. nucleic acid probes, capable of binding specifically to target molecules, e.g. target nucleic acid molecules and allowing a subsequent electrochemical detection of bound target nucleic acids.

The term "gravimetric detection" relates to methods which make use of a system that is able to detect changes in mass which occur e.g. when a probe binds its target.

The tumor cell marker genes as defined herein can be used for estimation of tumor cell content in breast cancer but may be useful also for other cancer types due to common genes indicative for stromal, vascular and immunological components of tumor tissue. Moreover, the tumor cell marker genes have been assigned to the chromosomal locations as described above covering genes which are highly informative for tumor cell content. On the basis of a large set of genome-wide gene expression microarray data from fresh-frozen breast cancer samples, chromosomal locations, and genes whose expression are associated with high or low tumor cell content where identified. The expression values of the identified tumor cell marker genes can be used alone or in combination with each other to estimate tumor cell content using a molecular approach as opposed to a visual analysis of histologic samples as used in the prior art.

The advantages of the methods for determining tumor cell content of the sample according to the present invention are (1) that an estimation of tumor cell content is possible in the molecular biological laboratory without the need for an experienced pathologist, (2) that tumor cell content can directly be measured in a multiplex gene expression array without a separate analysis and (3) that the molecularly assessed tumor cell content can easily be incorporated as a further variable into an algorithm for prognosis or prediction of a therapy response in order to improve the performance of a predictive or prognostic gene expression assay or test.

Routinely, in tumor diagnosis, tissue samples are taken as biopsies from a patient or are obtained as resected material in surgery and undergo diagnostic procedures. For this purpose, samples are fixed in formalin and/or paraffin and are then examined with e.g. immuno-histochemistry methods. The formalin treatment leads to inactivation of enzymes, as for example the RNA-digesting enzymes. For this reason, the mRNA status of the fixed tissue remains undigested. However, formalin treatment may lead to a partial depolymerisation of individual mRNA-molecules. For this reason, a preferred embodiment of the invention, total RNA is isolated from non-fixed samples, e.g. fresh samples, fresh-frozen samples, flash-frozen samples, or the like.

For determination of tumor cell content by visual inspection, HE stained histological sections are prepared. This can e.g. be done by preparing HE staining of paraffin embedded sections or cryo sections. Sections are prepared by microtome and HE staining according to standard protocols well known in the art. The HE staining method involves application of the basic dye hematoxylin, which colors basophilic structures with blue-purple hue, and alcohol-based acidic eosin Y, which colors eosinophilic structures bright pink. This staining allows differentiation of tumor cells from non-tumor cells based on cellular morphology.

It is preferred to use pairs of adjacent histological sections of the same sample for visual inspection and RNA Isolation, respectively.

RNA may be isolated by any known suitable method, e.g. using commercially available RNA isolation kits.

According to a preferred embodiment of the present invention, after lysis, the samples are treated withsilica-coated magnetic particles and a chaotropic salt, in order to purify nucleic acids contained in the sample for further analysis. This method of RNA isolation has been shown to yield satisfactory results even when RNA is extracted from fixed tissue samples. This method which allows successful purification of mRNA out of fixed tissue samples is disclosed in WO 030058649, WO 2006136314A1 and DE10201084A1, the content of which is incorporated herein by reference.

This RNA extraction method comprises the use of magnetic particles coated with silica (silicon dioxide, SiO₂). These highly pure magnetic particles coated with silica are used for isolating nucleic acids, including DNA and RNA, from cell and tissue samples; the particles may be retrieved from a sample matrix or sample solution by use of a magnetic field. These particles are particularly well-suited for the automatic purification of nucleic acids, mostly from biological samples for the purpose of detecting nucleic acids.

The selective binding of nucleic acids to the surface of these particles is due to the affinity of negatively charged nucleic acids to silica-containing media in the presence of chaotropic salt like guanidin isothiocyanat. The binding properties are for example described in EP 819696.

This approach is particularly useful for the purification of mRNA from formalin-fixated and/or paraffin-embedded (e.g. FFPE) tissue samples. In contrast to most other approaches which may result in small fragments that are not well suited for later gene expression analysis by PCR and/or hybridization technologies, this approach creates mRNA fragments which are large enough to allow such analysis.

This approach allows a highly specific determination of gene expression levels with one of the above-mentioned methods, in particular hybridization based methods, PCR-based method and/or array-based methods even in formalin-fixated and/or paraffin-embedded tissue samples and is therefore highly efficient in the context of the present invention, because it allows the use of fixed tissue samples which often are readily available in tissue banks and connected to clinical data bases, e.g. for follow-up studies to allow retrospective analysis.

### Example 1:

Total RNA was isolated from 130 fresh-frozen breast cancer samples with known tumor cell content ranging from 5% to 90%. Tumor cell content for these samples had been determined by a pathologist using visual inspection of stained immunohistological slides, and tumor cell content is expressed as proportion of tumor cells to the sum of all cells. Visual inspection was performed on histological sections of tissue samples, and RNA was isolated from sections of each sample.

Samples were discretized according to TCC into 2 groups: TCC < 33% (low TCC) and TCC > 66% (high TCC). The group with TCC < 33% was defined as "low TCC", the group with TCC > 66% was defined as "high TCC". The mRNA expression was investigated on Affymetrix U133A micro-array chips with post-processing of the data by using Affymetrix' standard MAS5 algorithm. Best informative genes for univariate separation of both groups were identified by t test statistics and a bootstrapping procedure. In a second step, the list of TCC marker genes was mapped with respect to chromosomal location and identified chromosomal locations which comprise clusters of genes whose expression are highly informative for tumor cell content.

### Example 2:

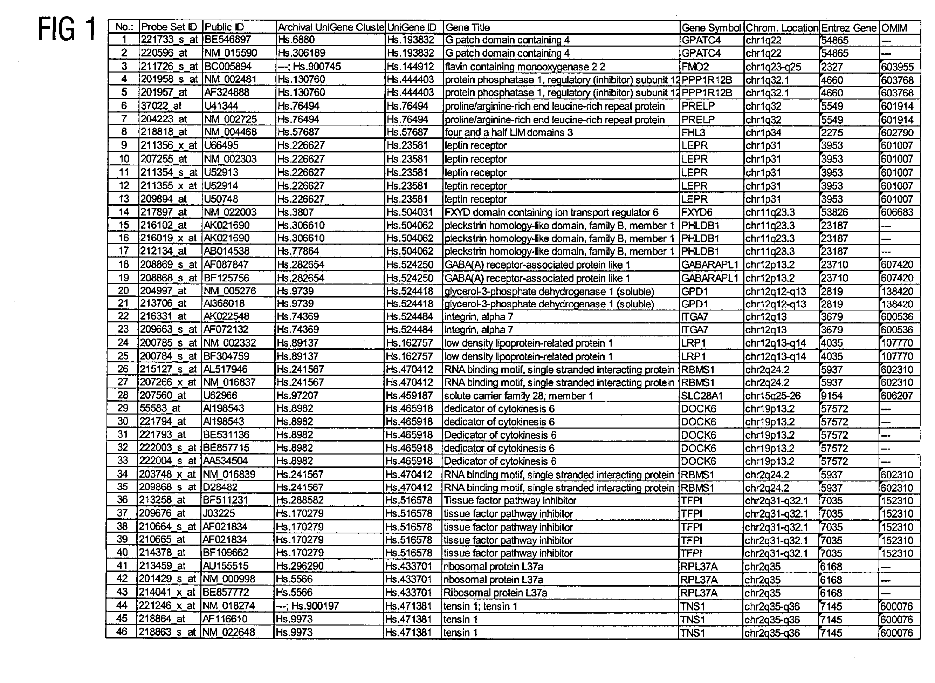

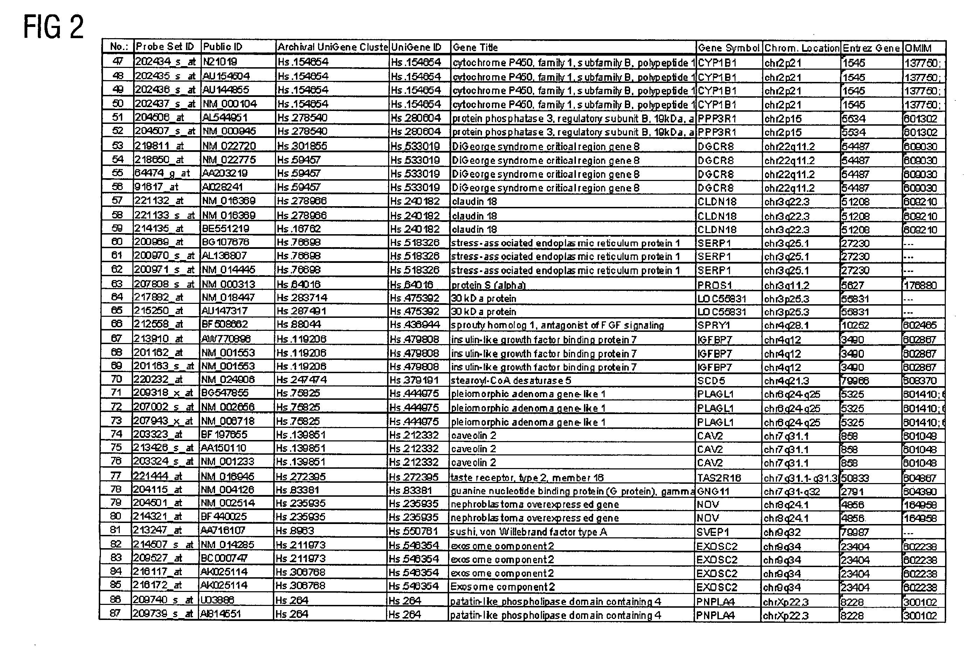

Table 1 shows a list of TCC marker genes identified by the above described method of example 1. Genes No. 1 to 87 are identifiable by their Affymetrix Probe set ID, the associated gene symbol, Public ID (= Accession Number) and common name. Figures 1 and 2 correspond to the list in table 1 and give additional information on TCC marker genes 1 to 87 including: Probe Set ID, Public ID, Archival UniGene Cluster, UniGene ID
Gene Title, Gene Symbol, Chromosomal Location, Entrez Gene (= Accession Number for Entrez PubMed), and OMIM.

In particular, it was found that TCC marker genes map to the following chromosomal locations: chr11q23.3, chr12p13.2, chr12q12-q13, chr12q13, chr12q13-q14, chr15q25-26, chr19p13.2, chr1p31, chr1p34, chr1q22, chr1q23-q25, chr1q32, chr1q32.1, chr22q11.2, chr2p15, chr2p21, chr2q24.2, chr2q31-q32.1, chr2q35, chr2q35-q36, chr3p25.3, chr3q11.2, chr3q22.3, chr3q25.1, chr4q12, chr4q21.3, chr4q28.1, chr6q24-q25, chr7q31.1, chr7q31.1-q31.3|7, chr7q31-q32, chr8q24.1, chr9q32, chrXp22.3, chrXq26.

In further tests, the following chromosomal locations have been shown to contain highly informative TCC marker genes identified by the method of the invention 1q22-32, 2p14-22, 3p11-25, 3q11-27, 4q12-28, 7q21-31, 9q31-34, 11q13-23, 12q13-14, 17q21-24.

### Example 3, 4, and 5:

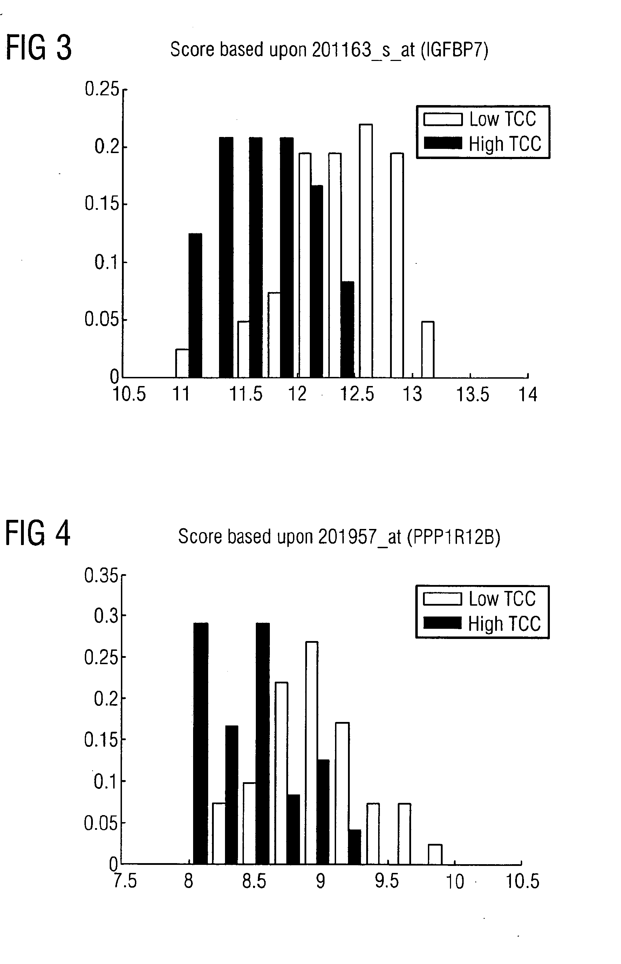

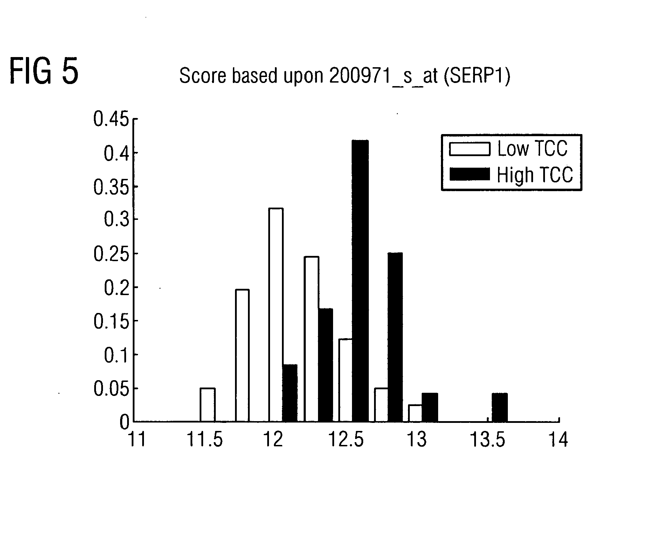

Figures 3, 4 and 5 show histograms depicting the separation of high tumor cell content and low tumor cell content samples according the expression level of three exemplary tumor cell content marker genes IGF PB7, PPP1R12B, and SERP1, respectively.

Analysis was performed according to the method described for example 1. Tumor cell content for these samples had been determined by a pathologist using visual inspection of stained immunohistological slides. Samples were separated into low TCC and high TCC samples as described above. Gene expression level of the respective genes was determined for each sample. The X axis in each histogram depicts a score of the log2-value of the expression value of the respective gene as determined on Affymetrix U133A microarray chips. For better visualization of results, the values were discretized into 10 equally large bins. The Y axis represents the percentage of samples allocated to each bin.

It can be seen that the three selected exemplary tumor cell content marker genes (IGFBP7, PPP1R12B, and SERP1, respectively) allow a good separation of the samples into high TCC and low TCC. It is thus shown that these genes are highly indicative of tumor cell content.

Determining the tumor cell content by measuring the expression level of a plurality of TCC marker genes (e.g. IGF PB7, PPP1R12B, and SERP1) and forming a new score based on the combined (e.g. average) expression level value of the plurality of TCC marker genes allows an even better separation. This points to the fact that using a combination of a plurality of TCC marker genes yields even more reliable results for tumor cell content of a sample.

**Table 1 (part 1): Probe Set IDs and corresponding TCC Marker Genes**

| No.: | Probe Set ID | Public ID | Gene Title | Gene Symbol |
|---|---|---|---|---|
| 1 | 221733_s_at | BE546897 | G patch domain containing 4 | GPATC4 |
| 2 | 220596_at | NM_015590 | G patch domain containing 4 | GPATC4 |
| 3 | 211726_s_at | BC005894 | flavin containing monooxygenase 2 | FM02 |
| 4 | 201958_s_at | NM_002481 | protein phosphatase 1, regulatory (inhibitor) subunit 12B | PPP1R12B |
| 5 | 201957_at | AF324888 | protein phosphatase 1, regulatory (inhibitor) subunit 12B | PPP1R12B |
| 6 | 37022_at | U41344 | proline/arginine-rich end leucine-rich repeat protein | PRELP |
| 7 | 204223_at | NM_002725 | proline/arginine-rich end leucine-rich repeat protein | PRELP |
| 8 | 218818_at | NM_004468 | four and a half LIM domains 3 | FHL3 |
| 9 | 211356_x_at | U66495 | leptin receptor | LEPR |
| 10 | 207255_at | NM_002303 | leptin receptor | LEPR |
| 11 | 211354_s_at | U52913 | leptin receptor | LEPR |
| 12 | 211355_x_at | U52914 | leptin receptor | LEPR |
| 13 | 209894_at | U50748 | leptin receptor | LEPR |
| 14 | 217897_at | NM_022003 | FXYD domain containing ion transport regulator 6 | FXYD6 |
| 15 | 216102_at | AK021690 | pleckstrin homology-like domain, family B, member 1 | PHLDB1 |
| 16 | 216019_x_at | AK021690 | pleckstrin homology-like domain, family B, member 1 | PHLDB1 |
| 17 | 212134_at | AB014538 | pleckstrin homology-like domain, family B, member 1 | PHLDB1 |
| 18 | 208869_s_at | AF087847 | GABA(A) receptor-associated protein like 1 | GABARAPL1 |
| 19 | 208868_s_at | BF125756 | GABA(A) receptor-associated protein like 1 | GABARAPL1 |
| 20 | 204997_at | NM_005276 | glycerol-3-phosphate dehydrogenase 1 (soluble) | GPD1 |
| 21 | 213706_at | AI368018 | glycerol-3-phosphate dehydrogenase 1 (soluble) | GPD1 |
| 22 | 216331_at | AK022548 | integrin, alpha 7 | ITGA7 |
| 23 | 209663_s_at | AF072132 | integrin, alpha 7 | ITGA7 |
| 24 | 200785_s_at | NM_002332 | low density lipoprotein-related protein 1 | LRP1 |
| 25 | 200784_s_at | BF304759 | low density lipoprotein-related protein 1 | LRP1 |
| 26 | 215127_s_at | AL517946 | RNA binding motif, single stranded interacting protein 1 | RBMS1 |
| 27 | 207266_x_at | NM_016837 | RNA binding motif, single stranded interacting protein 1 | RBMS1 |
| 28 | 207560_at | U62966 | solute carrier family 28, member 1 | SLC28A1 |
| 29 | 55583_at | AI198543 | dedicator of cytokinesis 6 | DOCK6 |
| 30 | 221794_at | AI198543 | dedicator of cytokinesis 6 | DOCK6 |
| 31 | 221793_at | BE531136 | Dedicator of cytokinesis 6 | DOCK6 |
| 32 | 222003_s_at | BE857715 | dedicator of cytokinesis 6 | DOCK6 |
| 33 | 222004_s_at | AA534504 | Dedicator of cytokinesis 6 | DOCK6 |
| 34 | 203748_x_at | NM_016839 | RNA binding motif, single stranded interacting protein 1 | RBMS1 |
| 35 | 209868_s_ at | D28482 | RNA binding motif, single stranded interacting protein 1 | RBMS1 |
| 36 | 213258_at | BF511231 | Tissue factor pathway inhibitor | TFPI |
| 37 | 209676_at | J03225 | Tissue factor pathway inhibitor | TFPI |
| 38 | 210664_s_at | AF021834 | Tissue factor pathway inhibitor | TFPI |
| 39 | 210665_at | AF021834 | Tissue factor pathway inhibitor | TFPI |
| 40 | 214378_at | BF109662 | Tissue factor pathway inhibitor | TFPI |
| 41 | 213459_at | AU155515 | ribosomal protein L37a | RPL37A |
| 42 | 201429_s_at | NM_000998 | ribosomal protein L37a | RPL37A |
| 43 | 214041_x_at | BE857772 | Ribosomal protein L37a | RPL37A |
| 44 | 221246_x_at | NM_018274 | tensin 1 /// tensin 1 | TNS1 |
| 45 | 218864_at | AF116610 | tensin 1 | TNS1 |
| 46 | 218863_s_at | NM_022648 | tensin 1 | TNS1 |

**Table 1 (part 2): Probe Set IDs and corresponding TCC Marker Genes**

| No.: | Probe Set ID | Public ID | Gene Title | Gene Symbol | Chrom. Location |
|---|---|---|---|---|---|
| 47 | 202434_s_at | N21019 | cytochrome P450, family 1, sub B, polypeptide 1 | CYP1B1 | chr2p21 |
| 48 | 202435_s_at | AU154504 | cytochrome P450, family 1, sub B, polypeptide 1 | CYP1B1 | chr2p21 |
| 49 | 202436_s_at | AU144855 | cytochrome P450, family 1, sub B, polypeptide 1 | CYP1B1 | chr2p21 |
| 50 | 202437_s_at | NM_000104 | cytochrome P450, family 1, sub B, polypeptide 1 | CYP1B1 | chr2p21 |
| 51 | 204506_at | AL544951 | protein phosphatase 3, regulatory subunit B | PPP3R1 | chr2p15 |
| 52 | 204507_s_at | NM_000945 | protein phosphatase 3, regulatory subunit B | PPP3R1 | chr2p15 |
| 53 | 219811_at | NM_022720 | DiGeorge syndrome critical region gene 8 | DGCR8 | chr22q11.2 |
| 54 | 218650_at | NM_022775 | DiGeorge syndrome critical region gene 8 | DGCR8 | chr12q11.2 |
| 55 | 64474_g_at | AA203219 | DiGeorge syndrome critical region gene 8 | DGCR8 | chr22q11.2 |
| 56 | 91617_at | AI028241 | DiGeorge syndrome critical region gene 8 | DGCR8 | chr22q11.2 |
| 57 | 221132_at | NM_016369 | claudin 18 | CLDN18 | chr3q22.3 |
| 58 | 221133_s_at | NM_016369 | claudin 18 | CLDN18 | chr3q22.3 |
| 59 | 214135_at | BE551219 | claudin 18 | CLDN18 | chr3q22.3 |
| 60 | 200969_at | BG107676 | stress-associated er protein 1 | SERP1 | chr3q25.1 |
| 61 | 200970_s_at | AL136807 | stress-associated er protein 1 | SERP1 | chr3q25.1 |
| 62 | 200971_s _at | NM_014445 | stress-associated er protein 1 | SERP1 | chr3q25.1 |
| 63 | 207808_s_at | NM_000313 | protein S (alpha) | PROS1 | chr3q11.2 |
| 64 | 217882_at | NM_018447 | 30 kDa protein | LOC55831 | chr3p25.3 |
| 65 | 215250_at | AU147317 | 30 kDa protein | LOC55831 | chr3p25.3 |
| 66 | 212558_at | BF508662 | sprouty homolog 1, antagonist of FGF signaling | SPRY1 | chr4q28.1 |
| 67 | 213910_at | AW770896 | insulin-like growth factor binding protein 7 | IGFBP7 | chr4q12 |
| 68 | 201162_at | NM_001553 | insulin-like growth factor binding protein 7 | IGFBP7 | chr4q12 |
| 69 | 201163_s_at | NM_001553 | insulin-like growth factor binding protein 7 | IGFBP7 | chr4q12 |
| 70 | 220232_at | NM_024906 | stearoyl-CoA desaturase 5 | SCD5 | chr4q21.3 |
| 71 | 209318_x_at | BG547855 | pleiomorphic adenoma gene-like 1 | PLAGL1 | chr6q24-q25 |
| 72 | 207002_s_at | NM_002656 | pleiomorphic adenoma gene-like 1 | PLAGL1 | chr6q24-q25 |
| 73 | 207943_x_at | NM_006718 | pleiomorphic adenoma gene-like 1 | PLAGL1 | chr6q24-q25 |
| 74 | 203323_at | BF197655 | caveolin 2 | CAV2 | chr7q31.1 |
| 75 | 213426_s_at | AA150110 | caveolin 2 | CAV2 | chr7q31.1 |
| 76 | 203324_s_at | NM_001233 | caveolin 2 | CAV2 | chr7q31.1 |
| 77 | 221444_at | NM_016945 | taste receptor, type 2, member 16 | TAS2R16 | chr7q31.1-q31.3 |
| 78 | 204115_at | NM_004126 | guanine nucleotide binding protein, gamma 11 | GNG11 | chr7q31-q32 |
| 79 | 204501_at | NM_002514 | nephroblastoma overexpressed gene | NOV | chr8q24.1 |
| 80 | 214321_at | BF440025 | nephroblastoma overexpressed gene | NOV | chr8q24.1 |
| 81 | 213247_at | AA716107 | sushi, von Willebrand factor type A | SVEP1 | chr9q32 |
| 82 | 214507_s_at | NM_014285 | exosome component 2 | EXOSC2 | chr9q34 |
| 83 | 209527_at | BC000747 | exosome component 2 | EXOSC2 | chr9q34 |
| 84 | 216117_at | AK025114 | exosome component 2 | EXOSC2 | chr9q34 |
| 85 | 216172_at | AK025114 | Exosome component 2 | EXOSC2 | chr9q34 |
| 86 | 209740_s_at | U03886pa | tatin-like phospholipase domain containing 4 | PNPLA4 | chrXp22.3 |
| 87 | 209739_s_at | AI814551 | patatin-like phospholipase domain containing 4 | PNPLA4 | chrXp22.3 |

References
1.) Cheung et al., Cancer 2004; 101:2303-8
2.) Cheung et al. Cancer 2002; 94:3042-8
3.) Schostak et al., BMC Urology 2006, 6:7
4.) Cui et al., World J Gastroenterol, 2001;7(3):381-386

## Claims

1. Method of determining the tumor cell content of a sample from a patient suffering from or at risk of developing a neoplastic disease, comprising the steps of:
a) isolating total RNA from the sample
b) determining the expression level of at least one tumor cell content (TCC) marker gene selected from the group consisting of the genes identified in table 1.

2. Method of determining the tumor cell content of a sample from a patient suffering from or at risk of developing a neoplastic disease, comprising the steps of:
a) isolating total RNA from the sample
b) determining the expression level of at least one tumor cell content (TCC) marker gene mapping to a chromosomal location selected from the group consisting of 1q22-32, 2p14-22, 3p11-25, 3q11-27, 4q12-28, 7q21-31, 9q31-34, 11q13-23, 12q13-14, 17q21-24.

3. Method according to any one of claims 1 or 2, wherein the expression level of 2 to 20 tumor cell content (TCC) marker genes is determined.

4. Method according to any one of claims 1 to 3 wherein the expression level of the at least one tumor cell content (TCC) marker gene is determined in relation to the expression level of at least one internal standard gene.

5. The method according to any one of claims 1 to 4, wherein the expression level is determined by one of the methods selected from the group consisting of
a) a hybridization-based method;
b) a PCR-based method;
c) determining the protein level, and
d) an array-based method.

6. Method according to any one of claims 1 to 5, comprising the additional step of correlating the tumor cell content with said patient's data, said data being selected from the group consisting of gene expression level of a further marker gene which is not a TCC marker gene according to claim 1 or 2, etiopathology data, clinical parameters, anamnesis data and/or data concerning the therapeutic regimen.

7. Method according to any of claims 1 to 6, wherein the sample is a sample containing breast cancer cells or suspected of containing breast cancer cells.

8. Method of quantifying an expression level of a gene in a biological sample, wherein the expression level of said gene in the sample is determined in relation to the tumor cell content as determined according to the method of any of claims 1 to 7.

9. A method of predicting a clinical outcome for a patient afflicted with a neoplastic disease, said method comprising the steps of:
a) obtaining a biological sample from said patient;
b) predicting from the tumor cell content of said sample, by the method according to any one of claims 1 to 7, therapeutic success for a plurality of individual modes of treatment; and
c) selecting a mode of treatment which is predicted to be successful in step (b).

10. Method according to claim 9, wherein an expression level of a further gene in the biological sample is determined in relation to the tumor cell content.

11. A kit useful for carrying out a method of any one of claims 1 to 10, comprising at least
a) a primer pair and/or a probe each having a sequence sufficiently complementary to a tumor cell content (TCC) marker gene selected from the group consisting the genes identified in table 1 and/or a tumor cell content (TCC) marker gene mapping to a chromosomal location selected from the group consisting of 1q22-32, 2p14-22, 3p11-25, 3q11-27, 4q12-28, 7q21-31, 9q31-34, 11q13-23, 12q13-14, 17q21-24; and/or
b) at least one antibody directed against a protein encoded by a tumor cell content (TCC) marker gene selected from the group consisting the genes identified in table 1 and/ or a tumor cell content (TCC) marker gene mapping to a chromosomal location selected from the group consisting of 1q22-32, 2p14-22, 3p11-25, 3q11-27, 4q12-28, 7q21-31, 9q31-34, 11q13-23, 12q13-14, 17q21-24.
